# EUROPÄISCHE PATENTSCHRIFT

(11) **EP 2 235 388 B1**
(45) Veröffentlichungstag und Bekanntmachung des Hinweises auf die Patenterteilung: **15.05.2013**
(21) Anmeldenummer: 09702659.5
(22) Anmeldetag: 20.01.2009
(51) Int. Cl.: F16B 25/00

(54) **SELBSTSCHNEIDENDER EINSCHRAUBKÖRPER MIT ZICKZACK-SPANNUTEN**
SELF-CUTTING SCREW-IN INSERT HAVING ZIGZAGGING FLUTES
CORPS À VISSER AUTOTARAUDEUR MUNI DE GOUJURES EN ZIGZAG

(30) Priorität: 20.01.2008 DE 102008005267
(43) Veröffentlichungstag der Anmeldung: 06.10.2010
(73) Patentinhaber: Hörmansdörfer, Gerd, 31303 Burgdorf-Beinhorn (DE)
(72) Erfinder: Hörmansdörfer, Gerd, 31303 Burgdorf-Beinhorn (DE)
(74) Vertreter: Jabbusch, Matthias
(86) Internationale Anmeldenummer: PCT/EP2009/000312
(87) Internationale Veröffentlichungsnummer: WO 2009/090103

(56) Entgegenhaltungen:
- WO-A-2004/042240
- DE-A1-102004 057 709
- DE-U1- 9 402 828

## Beschreibung

Die Erfindung betrifft einen selbstschneidenden Einschraubkörper mit einer vorzugsweise geknickten oder gekrümmten Mantelfläche und einem auf dieser Mantelfläche liegenden Bereich mit einem Gewinde, wobei sich dessen Gewindezahnprofil von der imaginären Achse des Einschraubkörpers weg - vorzugsweise subvertikal - erstreckt, und das Gewinde durch mindestens zwei Spannuten unterbrochen ist, gemäß den Ansprüchen 1 bis 5, wie z.B. einer einschraubbaren künstlichen Hüftgelenkpfanne und anderen Verkörperungen gemäß Anspruch 6, bei welchen auf Grund eines hin und her springenden Verlaufs der Spannut die zwischen Spannut und Gewindezahn gebildete Schneidkante wechselweise auf die eine Flanke des Gewindezahns oder die andere Flanke des Gewindezahns verlagert ist. Eine derartige Gestaltung ist für bestimmte Anwendungen vorteilhaft, weil dadurch die zum Einschrauben erforderlichen Kräfte ohne nachteilige Auswirkungen auf die sonstigen Eigenschaften des Einschraubkörpers merklich reduziert sind.

Gewinde sind als konstruktive Elemente des allgemeinen Maschinenbaus weit verbreitet. Gewöhnlich sind Gewinde zylinderförmig gestaltet. Daneben sind auch konische Gewinde z.B. für Ölfeldrohre in Gebrauch. Eine große Anzahl verschiedener Gewindeprofile ist bekannt und in Normen festgelegt. Üblicherweise ist das Gewindeprofil entlang seiner Erstreckung an einem Werkstück unveränderlich. Abweichend davon kann es aus herstellungstechnischen Gründen am Gewindeanfang bzw. -ende zu Abweichungen vom Nennprofil kommen. Es sind auch Varianten bekannt, bei denen eine zumindest in einem Teilbereich des Gewindes fließend sich ändernde Formgestalt des aus Gewinderille und Gewindezahn gebildeten Gewindeprofils realisiert ist, z.B. bei selbstschneidenden Schrauben, oder z.B. um das Einführen eines Schraubengewindes in ein Mutterngewinde zu erleichtern.

Spezielle geometrische Verhältnisse in Bezug auf das Gewinde bestehen jedoch vor allem bei Gewinden auf gekrümmten Flächen, wie sie insbesondere bei einschraubbaren künstlichen Hüftgelenkpfannen vorkommen. Hier sind hinsichtlich der Mantelform des Schalenkörpers z.B. hypo-, hemi-, oder hypersphärische, konischsphärische, parabolische toroidische, elliptische und ähnliche Geometrien bekannt. Bei spanenden Herstellungsverfahren für die Herstellung derartiger Schraubpfannen ergeben sich teilweise zwangsläufig fließend sich verändernde Verzerrungen des Gewindeprofils, welche in den meisten Fällen weder beabsichtigt noch erwünscht sind. Insbesondere bei der Benutzung von Gewindezähnen mit unsymmetrischen Flanken (den jeweiligen Seitenwinkeln des Gewindezahns) ergibt sich das Phänomen, dass je nach Kipprichtung des resultierenden Gewindezahns die Zahnhöhe vom Pfannenäquator in Richtung zum Pfannenpol hin fließend zu- bzw. abnimmt, woraus sich dann am polnahen Gewindeanfang entweder viel zu große oder nahezu verkümmerte Gewindezähne ergeben. Im ersten Fall führen die extrem großen Gewindezähne dazu, dass zum Einschrauben der Pfanne sehr hohe Kräfte erforderlich werden, bzw. das Implantat nicht bis zum vollständigen Knochenkontakt eingeschraubt werden kann. Im zweiten Fall wird lediglich eine sehr dürftige Primärfixation zu erreichen sein. In beiden Fällen besteht die Gefahr des Auslockerns des Implantats, welches als Konsequenz eine nochmalige Operation des Patienten bedeuten würde.

Man kann nun den Einfluss des Kippwinkels des Zahnprofils auf die Gewindezahnhöhe mehr oder weniger dadurch zurückdrängen, dass man ein subvertikal relativ zur imaginären Pfannenachse ausgerichtetes Zahnprofil verwendet. Bei Einschraubkörpern mit gekrümmter Mantelfläche bleibt jedoch ein weiteres Problem dadurch bestehen, dass sich die einzelnen Gewindezähne beim Einschrauben in eine entsprechend ausgeraffelte Kavität nicht - wie häufig angenommen - gleichzeitig oder gar mit dem in Einschraubrichtung vorn liegenden Gewindeanfang zuerst eingraben. Es ist vielmehr so, dass sich z.B. bei sphärischen Hüftgelenkpfannen mit vier Gewinderippenumläufen der vom Äquator aus gesehen zweite Umlauf zuerst eingräbt, gefolgt vom ersten und dritten Umlauf. Erst danach, wenn sich die genannten drei Umläufe bereits weitgehend eingegraben haben, kommt der vierte Umlauf in Kontakt mit der Kavität. Weil nun die Zähne des ersten bis dritten Umlaufs in der bereits geschnittenen Rille zwangsweise geführt sind, unterliegt der vierte Gewindeumlauf hinsichtlich seiner Eindringrichtung diesem Zwang. Bei einem subvertikal stehenden Gewindezahnprofil entsteht dann ein radial nach außen gerichtetes Schieben welches ohne das Vorhandensein von Spannuten auf beiden Flanken des Gewindezahnprofils zu Klemmkräften führt.

Wenn nun ein derartiges Gewinde zwecks Erzielung eines selbstschneidenden Verhaltens mit Spannuten ausgestattet werden soll, so wird gemäß dem Stand der Technik entweder auf einen geraden oder einen schrägen Verlauf zurückgegriffen. Bei einem geraden Verlauf beträgt dann der Schrägungswinkel 0°, oder kann als Drallwinkel von 0° definiert werden. Die dabei am Gewindezahn gebildete Schneid-oder Spanfläche steht dann neutral im Raum, woraus ein schlechtes Schneidverhalten resultiert. Ein besseres Ergebnis ist mit einem schrägen Verlauf der einzelnen Spannuten erzielbar. Hier sind Schrägungswinkel oder auch Drallwinkel zwischen +45° bis -45° bekannt, allerdings jeweils mit einheitlicher Schrägungsrichtung oder einheitlicher Drallrichtung. Dadurch ist an einem Ende des Gewindezahns bezüglich jeweils einer der beiden Gewindezahnflanken eine so genannte positiv stehende Schneid- oder Spanfläche gebildet. Die Schneidkante dieser Schneid- oder Spanfläche liegt dann immer auf der gleichen Gewindezahnflanke und sorgt dort während des Vorschubs für einen effektiven Schnitt. Dieser Effekt tritt an der gegenüber liegenden Gewindeflanke nicht auf, weil hier nur ein stumpfer Winkel zwischen der Gewindezahnflanke und der Schneid- oder Spanfläche gebildet ist. Dementsprechend treten an dieser Gewindezahnflanke ausschließlich Quetsch- und Verdrängungskräfte auf. Ein unerwünscht hoher Wert des Einschraubdrehmoments ist die Folge dieses Problems.

Im Bereich der Technik sind rechts gedrallte Gewinde Standard. Links gedrallte Gewinde sind besonderen Anwendungen vorbehalten, z.B. wenn bei einer entsprechend gerichteten Torsionsbeanpruchung eine Lockerung der Gewindeverbindung zu erwarten ist. Dem gemäß ist allgemein die Erwartungshaltung tief verwurzelt, eine Schraubverbindung rechtsgängig festzuziehen und linksgängig zu lösen. Alle Schraubdeckel und Schraubkappen sind derart gestaltet, auch z.B. selbstschneidende Schrauben. Bei Einschraubkörpern (z.B. künstlichen Hüftgelenkpfannen) ist das nicht anders. Sind Einschraubkörper durch Spannuten selbstschneidend ausgelegt, so verlaufen diese Spannuten häufig neutral, also mit einem Drallwinkel von null Grad. Der Querschnitt des jeweiligen Gewindezahns bildet dann eine Schneid- oder Spanfläche, welche wegen des Steigungswinkels des Gewindes ganz leicht positiv steht. Manche selbstschneidenden Blechschrauben besitzen leicht links gedrallte oder leicht schräg nach links verlaufende Spannuten, die allerdings lediglich den Steigungswinkel des Gewindes kompensieren und dann mit ihrer Schneid- oder Spanfläche neutral positioniert sind. Links gedrallte Spannuten sind zum Beispiel bei Gewindebohrern bekannt, welche bei Durchgangsgewinden die beim Schneiden anfallenden Späne vom Bohrer weg durch das Gewindeloch hindurch abtransportieren sollen.

Bei künstlichen Hüftgelenkpfannen sind bislang ausschließlich Rechtsgewinde bekannt. Die Gewindezähne stehen bei den meisten Produkten vertikal zur Pfannenachse. Wenn diese Hüftgelenkpfannen eine gekrümmte oder geknickte Mantelkontur, gerade verlaufende Spannuten und eine konstante Gewindesteigung besitzen, dann sind die Kräfte während des Einschraubvorgangs an der durch die Spannuten an den Gewindezähnen gebildeten Schneid- oder Spanflächen und deren mehr oder weniger neutral stehenden Schneidkanten zumindest theoretisch gleichmäßig verteilt. Wegen der vom Operateur in axialer Richtung während des Einschraubens ausgeübten Andruckkraft (geschätzt: ca. 50 bis 100 N) verlagert sich die Schneidkraft jedoch mehr auf die polwärts liegende Schneidkante des Gewindezahns. Es ist dann überaus sinnvoll, diese Seite mit einer Schneidkante mit positivem Schneid-oder Spanwinkel zu gestalten, weil damit der Einschraubkraftbedarf etwas kleiner wird. Dieses ist in einfacher Weise durch eine rechtsgerichtete oder rechts gedrallte Spannut mit ausgeprägtem Schrägungs- oder Drallwinkel realisierbar.

Mit der oben beschriebenen Ausführung ist allerdings das bezüglich des Einschraubverhaltens technisch mögliche Optimum nicht erreichbar. In dem Maße nämlich, um das der Spanwinkel größer - und damit positiver wird - wird die andere Schneidkante der Schneid- oder Spanfläche stumpfer - und damit negativer. Ohne einen Freiwinkel am Kopf bzw. an den Flanken des Gewindezahns, und ohne Exposition vorhandener Schneidkanten muss immer von einem überhöhten Einschraubkraftbedarf und dem Auftreten von körnenden Effekten am Rezipienten auszugehen sein. Dadurch wird auch die Überdrehreserve nachteilig reduziert.

Es sind bislang drei künstliche Hüftgelenkpfannen mit linksgerichteter Spannut bei gekrümmter Mantelfläche und neutral stehenden Gewindezahn auf den Markt gekommen. Es sind dies das Modell MC der Firma Brehm, das Modell MT der Firma Zimmer, sowie das Modell PAC der Firma DePuy. Bei der ersteren beträgt der Schrägungswinkel 15°, bei den anderen beiden 10°. Der Spanwinkel der hauptsächlich wirkenden Schneidkante ist dann stumpf (Fachbezeichnung "negativ") und führt daher zu einem ungünstig klemmenden, körnenden und insgesamt nicht akzeptablen Schneidverhalten.

Es bestand daher die Aufgabe zur Schaffung von vorzugsweise spanend auf entsprechend ausgerüsteten CNC-Maschinen herstellbaren Einschraubkörpern (z.B. in Gestalt von künstlichen Hüftgelenkpfannen, Schenkelhalsschrauben, Schulter-oder Zahnimplantaten und dergleichen) mit einem mindestens in einem Teilbereich auf einer gekrümmten oder abgewinkelten Mantelfläche liegenden Gewinde mit subvertikal zu der imaginären Achse des Einschraubkörpers ausgerichtetem Gewindezahnprofil mit mindestens zwei Spannuten und einem gegenüber herkömmlichen Einschraubkörpern reduzierten Einschraubdrehmoment.

Die Aufgabe wird nach der Erfindung durch die Zurverfügungstellung eines speziellen selbstschneidenden Einschraubkörpers mit einer vorzugsweise mindestens partiell geknickten oder gekrümmten Mantelfläche und einem auf dieser Mantelfläche liegenden Bereich mit einem Gewinde mit einem von der imaginären Achse des Einschraubkörpers weg ausgerichtetem Gewindezahnprofil, und einem durch mindestens zwei Spannuten unterbrochenen Gewinderippenzug, gemäß den Ansprüchen 1 bis 5 gelöst, wie z.B. einer einschraubbaren künstlichen Hüftgelenkpfanne und anderen Verkörperungen gemäß Anspruch 6, bei welchem die Schrägungsrichtung oder die Drallrichtung der Spannuten zwischen einem rechts- und linksweisenden Verlauf hin und her wechselt, wobei die jeweilige Schneidkante in ihrer Lage ständig zwischen der rechten und linken Gewindezahnflanke verlagert ist.

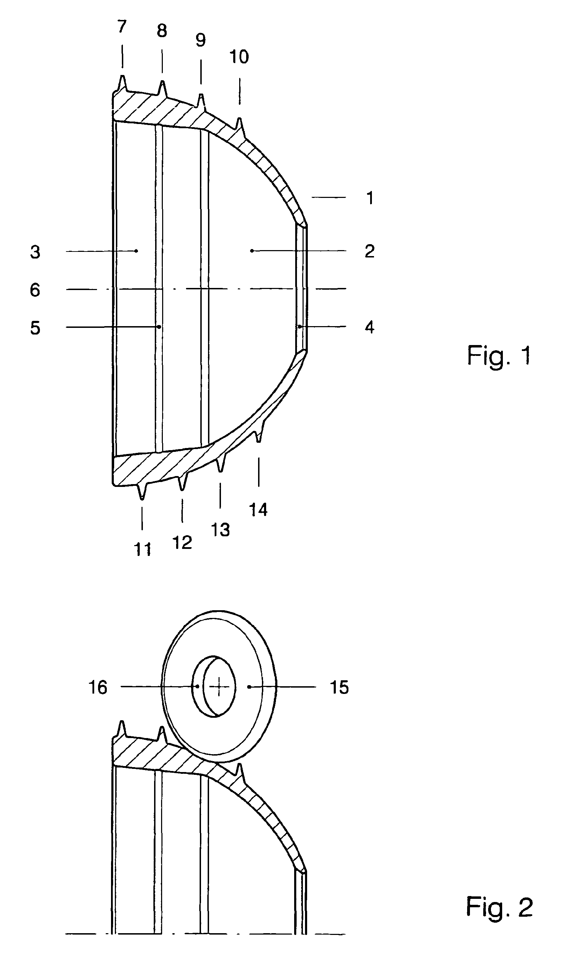

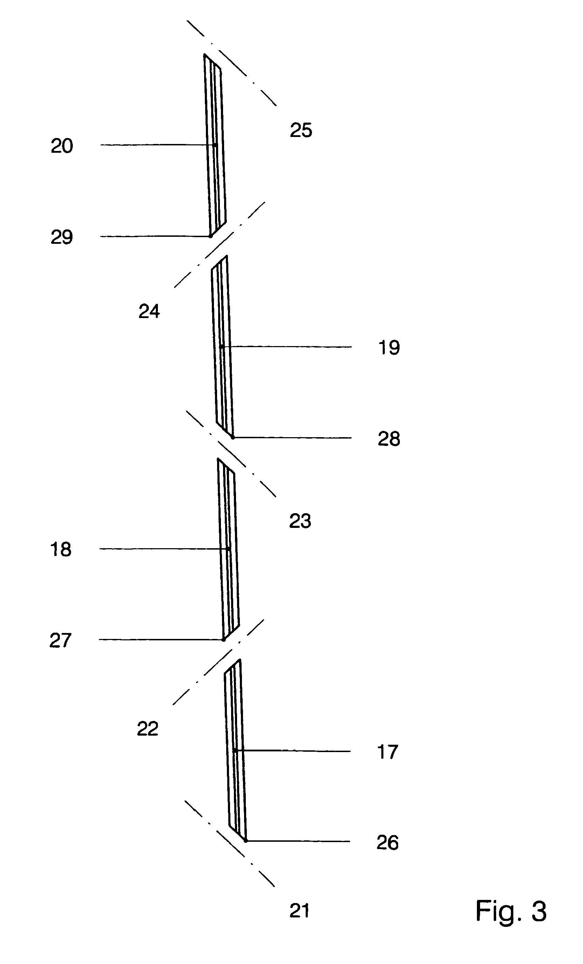

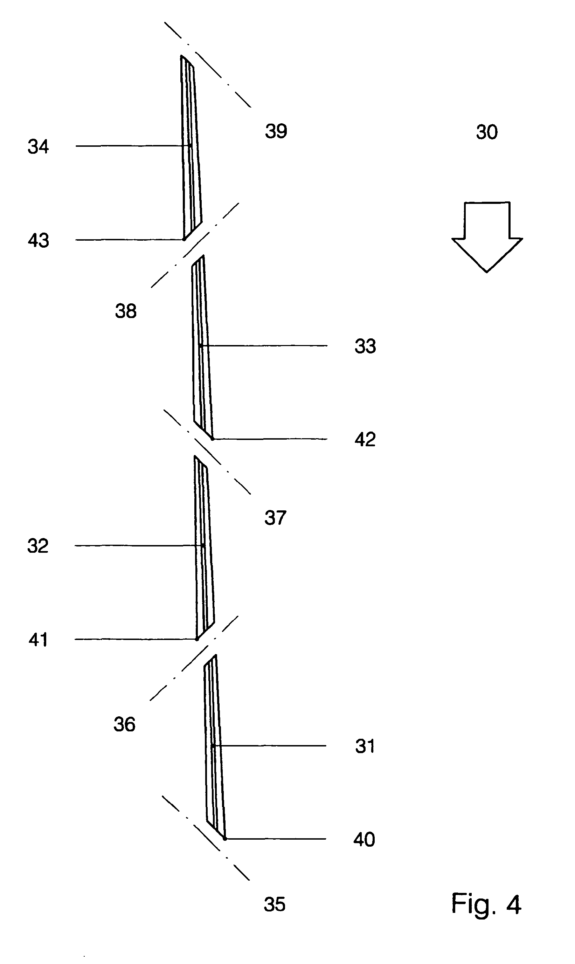

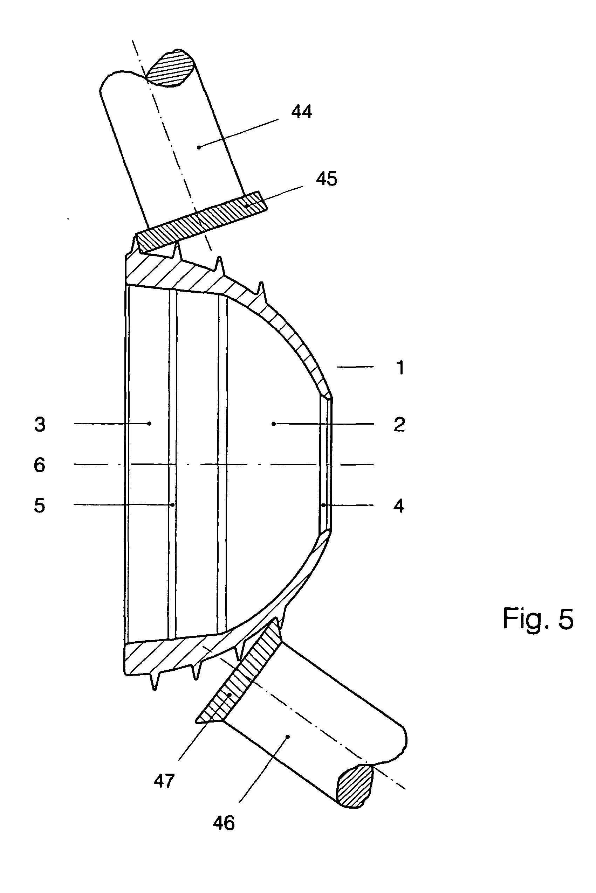

Zum besseren Verständnis soll die Erfindung im Folgenden anhand von fünf Zeichnungsfiguren näher erläutert werden. Als Beispiel eines Einschraubkörpers mit gekrümmter Mantelfläche wurde dabei auf eine vereinfacht gezeichnete Hüftgelenkpfanne und ein schematisch dargestelltes Teilstück eines Gewinderippenzuges zurückgegriffen. Fig. 1 zeigt das Schnittbild einer künstlichen Hüftgelenkpfanne mit Gewinde gemäß dem Stand der Technik. In **Fig. 2** wird eine Möglichkeit der spanenden Herstellung einer Spannut mit negativem Schrägungswinkel an einer der **Fig.1** entsprechenden Hüftgelenkpfanne gezeigt. **Fig. 3** und **Fig. 4** stellen das Teilstück eines Gewinderippenzuges dar, davon **Fig. 4** eine anspruchsvollere Variante. **Fig. 5** zeigt das Beispiel einer frästechnischen Bearbeitung der Zahnflanken.

Die **Fig. 1** zeigt die schematische Darstellung einer geschnittenen künstlichen Hüftgelenkpfanne **1** der Größe 52 gemäß dem Stand der Technik in einem im Verhältnis von 2:1 vergrößertem Maßstab. Das gezeigte Beispiel besitzt eine sphärische Kontur des gekrümmten Schalenmantels. Es sind oben vier Gewindezähne **7, 8, 9** und **10** eines eingängigen Gewindes zu sehen, und unten **11, 12, 13** und **14.** Es wird ein produktionstechnischer Zustand vor Einbringung der Spannuten gezeigt. Die imaginäre Mittelachse der Hüftgelenkpfanne wird durch eine strich-punktierte Linie **6** angedeutet. Die innere Geometrie ist durch einen sphärischen **2** und einen konischen Bereich **3** gekennzeichnet. Ferner ist eine Schnapprille **5** zur Einrastung des Inletts und ein Bodenloch **4** vorhanden.

In der **Fig. 2** ist die aus **Fig. 1** übernommene und nun halbierte künstliche Hüftgelenkpfanne dargestellt. Auf Grund der Darstellungsweise sind keine Spannuten zu sehen. Der aus vier Umläufen bestehende Gewinderippenzug besitzt ein symmetrisches Zahnprofil. Es weist einen eingeschlossenen Flankenwinkel von 24° auf und steht senkrecht auf der imaginären Pfannenachse. Dem gemäß betragen die Teilflankenwinkel des Gewindeprofils -12° und +12° für die jeweilige Flanke des Gewindezahns. Bei einer Höhe des Gewindezahns von nur 2 mm wurde für die Gewindesteigung ein Wert von 5 mm gewählt. Dieser relativ hohe Wert ist aus mehreren Gründen besonders vorteilhaft. Zum einen berücksichtigt er das Festigkeitsverhältnis zwischen dem Werkstoff für das Implantat und dem aufnehmenden "Werkstoff" Knochen. Zum anderen bewirkt die dadurch niedrige Zahl an Gewinderippenumläufen ein deutlich verkleinertes Einschraubdrehmoment. Schließlich wird dadurch noch Platz geschaffen, um mit dem die Spannuten fräsenden oder schleifenden Werkzeug in die Gewinderippe und wahlweise auch in die Mantelfläche der Hüftgelenkpfanne eintauchen zu können, ohne die benachbarten Gewindezähne zu beschädigen. In der Praxis werden solche Eintauchtiefen zwischen 0 und 1 mm realisiert und dazu Scheibenfräser, z.B. konvexe Halbkreisformfräser **15** oder auch Winkelstirnfräser oder dergleichen mit geeigneter Bohrung **16** für die Aufnahme auf einem Fräsdorn verwendet. Der in der **Fig. 2** gezeigte Scheibenfräser ist um **45°** gegen die Axialebene geschwenkt, um die erfindungsgemäße Schrägung der Spannut zu realisieren. Bei der Bearbeitung einer einzelnen derartigen Spannut mit z.B. einem positiven Schrägungs- oder Drallwinkel folgt dem gemäß bei der Bearbeitung der rechts und links benachbarten Spannuten eine winkelmäßig (im Beispiel um 90°) versetzte Positionierung des Werkzeugs, sodass daraus ein Schrägungs- oder Drallwinkel mit geändertem Vorzeichen resultiert.

Das erfindungsgemäße Ergebnis einer Bearbeitung eines Gewinderippenzuges wird in **Fig. 3** anhand eines schematischen Beispiels gezeigt. Es sind vier abgewickelte Gewindezähne **17, 18, 19, 20** mit einem aus **Fig. 1** und **Fig. 2** übernommenen Gewindezahnprofil dargestellt, welche durch die Einbringung von Spannuten **21, 22, 23, 24, 25** (angedeutet durch strich-punktierte Linien), entstanden sind. Der schräge Verlauf des Gewinderippenzuges soll den Einfluss der Gewindesteigung andeuten. Auf eine exakte Abbildung der in der Praxis auftretenden Verzerrungen wegen der Positionierung auf einer gekrümmten oder geknickten Mantelfläche des Einschraubkörpers wurde wegen des hohen zeichnerischen Aufwands verzichtet. Die Zeichnungsfigur verdeutlicht den ständigen Umsprung zwischen einem rechts- und einem linksgerichteten Verlauf der aufeinander folgenden Spannuten, wodurch die gebildeten Schneidkanten **26, 27, 28, 29** wechselweise auf die eine oder die andere Flanke des Gewindezahns verlagert sind. Dabei ist der winkelmäßige Verlauf der Spannuten untereinander bzw. relativ zum Steigungswinkel des Gewinderippenzuges unkritisch, so lange ein möglichst positiver Spanwinkel realisiert ist. Vorzugsweise betrifft die jeweilige Spannut lediglich einen einzigen Gewindezahn und erstreckt sich in ihrer jeweiligen Länge jeweils bis in den Bereich der benachbarten Gewinderillen hinein.

Eine der **Fig. 3** entsprechende Gestaltung des Gewindes wird bei einer relativ zur imaginären Achse eines Einschraubkörpers subvertikalen Positionierung des Gewindezahnprofils gegenüber konventionellen Ausführungen zu einem merklich reduzierten Einschraubdrehmoment führen. Dieses Verhalten wird jedoch gemäß der Erfindung weit übertroffen, wenn die jeweiligen Schneidkanten der einzelnen Zähne aus dem Verlauf des Gewinderippenzuges heraus exponiert und/oder hinter der Schneidkante mit einem Freiwinkel gestaltet werden. Ein derartiges Konzept wird in **Fig. 4** gezeigt.

Der in **Fig. 4** wie zuvor abgewickelt dargestellte Gewinderippenzug wird aus den vier Gewindezähnen (auch bezeichnet als Gewindeflügel) **31, 32, 33, 34** gebildet, welche jeweils durch strich-punktiert angedeutete Spannuten **35, 36, 37, 38,** 39 begrenzt sind. Diese Spannuten verlaufen abwechselnd in rechter und linker Richtung, wofür im gezeigten Beispiel ein Winkel von jeweils +45° und -45° zur imaginären Achse des Einschraubkörpers gewählt wurde. An den stirnseitigen Schneid- oder Spanflächen der Gewindezähne sind jeweils Schneidkanten **40, 41, 42, 43** vorhanden, welche wegen des schrägen Verlaufs der Spannuten hinter sich so genannte positive Spanwinkel besitzen. Diese Schneidkanten nehmen bezüglich ihrer Seitenlage auf dem Gewindezahn jeweils alternierende Stellungen ein. Dem gemäß liegt die Schneidkante **40** in der Zeichnung auf der rechten Flanke des Gewindezahns, die Schneidkante **41** auf der linken Seite, die Schneidkante **42** wiederum auf der rechten, und so weiter. Der Zeichnungsfigur ist gut zu entnehmen, dass die Breite des jeweiligen Gewindezahns, und damit auch dessen Höhe, an der in Einschraubrichtung vorn liegenden Stirn (Spanfläche) gegenüber dem Ende des Gewindezahns deutlich vergrößert ist. Während nun die in der Zeichnung rechts liegenden Flanken der Gewindezähne **31** und **32** miteinander fluchten, steht die Schneidkante **41** des Gewindeflügels **32** nach links gegenüber dem Ende des Gewindeflügels **31** heraus. Die Auslegung des Gewindes ist dabei an die Einschraubrichtung gebunden. Diese ist mit dem Pfeil **30** gekennzeichnet.

Das Beispiel einer Herstellungsmöglichkeit für die erfindungsgemäßen Zahnflanken mit speziellem Verlauf **z. B.** in Form von Schraubflächen oder mit Freiwinkeln, insbesondere jedoch mit hin-und-her springendem Verlauf wird in **Fig. 5** gezeigt. Dabei wurden die Kennziffern 1 bis 6 aus der **Fig. 1** übernommen. Ein erster Fräser **45** auf einem Schaft **44** ist für die Bearbeitung der polseitigen Zahnflanke bestimmt, während ein zweiter Fräser **47** auf einem Schaft **46** für die Bearbeitung der äquatorseitigen Zahnflanke vorgesehen ist. Eine elegante und Bearbeitungszeit sparende Herstellungsmethode für das vorgeschlagene Gewinde steht mit dem so genannten Humpeldrehen zu Verfügung, welches z. B. aus der EP 1 051 131 bekannt ist. Die frästechnisch oder per Humpeldrehen realisierten Bearbeitungsschritte sollten vorzugsweise lediglich der Endbearbeitung dienen, nachdem zuvor ein Großteil des zwischen den Zähnen zu zerspanenden Volumens bereits drehtechnisch ausgeräumt worden ist.

Es versteht sich, dass die Erfindung in einer großen Zahl von praktischen Verkörperungen umsetzbar ist, welche deutlich über die in den Figuren gezeigte Bandbreite hinausgeht. Insbesondere das Gewinde des Einschraubkörpers erlaubt eine recht breite Auslegung. Diese besteht z.B. in der Wahl der Gangzahl, des Gewindeprofils, dessen Höhe und Breite, dem eingeschlossenen Flankenwinkel und dem Kippwinkel, bzw. den Teilflankenwinkeln, der Gewindesteigung, auch einer variierenden Gewindesteigung, dem Span- und den Freiwinkeln sowie der Form, Breite, Tiefe und dem Schrägungs- oder Drallwinkel der Spannuten und deren Anzahl. So können z.B. die Gewindezähne aus Schraubflächen gebildet sein, oder durch ein beginnend von der Spanfläche aus in Richtung zum Gewindezahnende hin sich ausdünnendes Gewindeprofi mit reduzierter Breite und/oder Höhe des Gewindezahnprofils.

Mit der Erfindung wird somit ein vielseitig umsetzbarer Einschraubkörper mit selbstschneidendem Gewinde und einer im Gewindebereich liegenden, vorzugsweise gekrümmten und/oder geknickten Mantelfläche und subvertikal auf der imaginären Achse des Einschraubkörpers stehendem Gewindezahnprofil zur Verfügung gestellt, welcher aufgrund seiner speziellen Gestaltung gegenüber bisherigen Ausführungen über ein deutlich verbessertes Einschraub- und Überdrehverhalten verfügt und von dem z.B. im Falle von Implantaten eine Reduzierung der Lockerungsrate erwartet werden kann. Der erfindungsgemäße Einschraubkörper ist ohne technische Probleme und ohne zeitlichen Mehraufwand auf numerisch gesteuerten Maschinen herstellbar.

## Patentansprüche

1. Selbstschneidender Einschraubkörper mit einer vorzugsweise geknickten und / oder gekrümmten Mantelfläche und einem auf dieser Mantelfläche liegenden Bereich mit einem Gewinde, wobei sich dessen Gewindezahn mit seinem Gewindezahnprofil von der imaginären Achse des Einschraubkörpers weg erstreckt, und das Gewinde durch mindestens zwei schräge oder gedrallte Spannuten unterbrochen ist, **dadurch gekennzeichnet, dass** auf Grund eines zwischen rechts- und links gerichteten hin und her springenden Verlaufs der Spannut die zwischen Spannut und Gewindezahnflanke gebildete Schneidkante wechselweise auf die eine oder die andere Flanke des Gewindezahns verlagert ist.

2. Einschraubkörper gemäß Anspruch 1, **dadurch gekennzeichnet, dass** das Gewindezahnprofil senkrecht zur imaginären Achse des Einschraubkörpers ausgerichtet ist.

3. Einschraubkörper gemäß Anspruch 1 oder 2, **dadurch gekennzeichnet, dass** die Schneidkante aus dem Verlauf des Gewinderippenzuges heraus exponiert ist, das heißt, dass die Schneidkante des Gewindeflügels zur einen Seite gegenüber dem Ende des in Einschraubrichtung vorauslaufenden Gewindeflügels heraussteht, während die auf der anderen Seite liegenden Flanken der Gewindezähne miteinander fluchten.

4. Einschraubkörper gemäß einem der Ansprüche 1 bis 3, **dadurch gekennzeichnet, dass** die den jeweiligen Gewindezahn einhüllenden Flächen Ausschnitte aus Schraubflächen sind.

5. Einschraubkörper gemäß einem der Ansprüche 1 bis 3, **dadurch gekennzeichnet, dass** mindestens eine der drei den jeweiligen Gewindezahn einhüllenden Flächen hinter der Spanfläche einen Freiwinkel bildet.

6. Einschraubkörper gemäß einem der Ansprüche 1 bis 5, **dadurch gekennzeichnet, dass** er als Implantat, insbesondere als künstliche Hüftgelenkpfanne, Teil eines künstlichen Schultergelenks, Schenkelhalsschraube, Knochenschraube oder Zahnimplantat realisiert ist.

## Claims

1. A self-cutting screwed body with a preferably bent and/or curved jacket surface and an area with a thread lying on this jacket surface, wherein the threaded tooth profile of its threaded tooth extends away from the imaginary axis of the screwed body, and the thread is interrupted by at least two inclined or twisted chip flutes, **characterized in that**, because the chip flute jumps back and forth to the right and left, the cutting edge formed between the chip flute and threaded tooth flank is alternately displaced on one or the other flank of the threaded tooth.

2. The screwed body according to claim 1, **characterized in that** the threaded tooth profile is aligned perpendicular to the imaginary axis of the screwed body.

3. The screwed body according to claim 1 or 2, **characterized in that** the cutting edge is exposed out of the progression of the threaded ridge tension, meaning that the cutting edge of the threaded wing projects to one side relative to the end of the threaded wing running ahead in the screw-in direction, while the flanks of the threaded teeth lying on the other side are flush with each other.

4. The screwed body according to one of claims 1 to 3, **characterized in that** the surfaces sheathing the respective threaded tooth are cutouts from the screw surfaces.

5. The screwed body according to one of claims 1 to 3, **characterized in that** at least one of the three surfaces sheathing the respective threaded tooth forms a free angel behind the chip surface.

6. The screwed body according to one of claims 1 to 5, **characterized in that** it is realized as an implant, in particular as an artificial acetabular fossa, part of an artificial shoulder joint, femoral head screw, bone screw or tooth implant.

## Revendications

1. Corps à visser autotaraudeur avec une surface d'enveloppe de préférence coudée et/ou courbée et avec une zone située sur cette surface d'enveloppe avec un filetage pour lequel la dent de filetage de celui-ci avec son profil de dent de filetage s'étend en s'éloignant de l'axe imaginaire du corps à visser et le filetage est interrompu par au moins deux goujures obliques ou hélicoïdales **caractérisé en ce qu'**en raison d'un tracé à saut en va-et-vient orienté entre la gauche et la droite de la goujure, l'arête de coupe formée entre la goujure et le flanc de dent de filetage est déplacée alternativement sur l'un ou sur l'autre flanc de la dent de filetage.

2. Corps à visser selon la revendication 1 **caractérisé en ce que** le profil de dent de filetage est orienté perpendiculairement à un axe imaginaire du corps à visser.

3. Corps à visser selon la revendication 1 ou 2 **caractérisé en ce que** l'arête de coupe est exposée en dehors de par le tracé du train des nervures de filetage, c'est-à-dire que l'arête de coupe de l'aile de filetage dépasse d'un côté opposé à l'extrémité de l'aile de filetage précédente dans le sens de vissage, tandis que les flancs des dents de filetage situés de l'autre côté sont alignés entre eux.

4. Corps à visser selon une quelconque des revendications 1 à 3 **caractérisé en ce que** les surfaces enveloppant la dent de filetage respective sont des découpes des surfaces de vissage.

5. Corps à visser selon une quelconque des revendications 1 à 3 **caractérisé en ce qu'**au moins une des trois surfaces enveloppant la dent de filetage respective forme une angle de dépouille derrière la surface de coupe.

6. Corps à visser selon une quelconque des revendications 1 à 5 **caractérisé en ce qu**'il est réalisé comme implant, en particulier comme cavité cotyloïde artificielle, partie d'une articulation scapulohumérale artificielle, vis de col de fémur, vis pour fracture osseuse ou implant dentaire.
